# EUROPEAN PATENT APPLICATION

(11) **EP 4 827 253 A1**
(43) Date of publication of application: **21.10.2026**
(21) Application number: 24914921.2
(22) Date of filing: 29.09.2024
(51) Int. Cl.: H05K 7/14, H05K 9/00

(54) **CIRCUIT BOARD COMPONENT AND ELECTRONIC DEVICE**

(30) Priority: 02.01.2024 CN 202420021841 U
(71) Applicant: Huawei Technologies Co., Ltd., Shenzhen, Guangdong 518129 (CN)
(72) Inventor: ZOU, Bin, Shenzhen, Guangdong 518129 (CN); ZHAO, Menglong, Shenzhen, Guangdong 518129 (CN)
(74) Representative: Pfenning, Meinig & Partner mbB
(86) International application number: PCT/CN2024/122565
(87) International publication number: WO 2025/145700

(57) **Abstract**

This application provides a circuit board assembly (100) and an electronic device (1000). An electromagnetic shielding member (120) is disposed on a mainboard (110), a detection module (130) may be disposed on the electromagnetic shielding member (120) as an independent module, the mainboard (110), the electromagnetic shielding member (120), and the detection module (130) are stacked and assembled to form the circuit board assembly (100), the circuit board assembly (100) is assembled on a middle frame (300), and a bottom housing (200) is mounted on the middle frame (300). The detection module (130) is electrically connected to the mainboard (110) to implement signal transmission. A safety gap may not be reserved between the electromagnetic shielding member (120) and the detection module (130), and a support with a specific thickness may not be disposed on the bottom housing (200). The electronic device (1000) in embodiments can be miniaturized, and an overall thickness is small, allowing the entire device to be thinned by more than 0.15 mm. When being dropped or squeezed in a production process or a user use scenario, the detection module (130), the electromagnetic shielding member (120), and the mainboard (110) undergo synchronous micro-deformation, to reduce a failure or damage caused by collision of the detection module (130). The electronic device (1000) in embodiments is easy to assemble, and an assembly process is simple.

## Description

This application claims priority to Chinese Patent Application No. 202420021841.X, filed with the China National Intellectual Property Administration on January 2, 2024 and entitled "CIRCUIT BOARD ASSEMBLY AND ELECTRONIC DEVICE", which is incorporated herein by reference in its entirety.

### TECHNICAL FIELD

Embodiments of this application relate to the field of circuit board assembly technologies, and in particular, to a circuit board assembly and an electronic device.

### BACKGROUND

An electronic device like a watch or a band usually includes components such as a middle frame, a bottom housing, a mainboard, and a detection module. The detection module (for example, photoplethysmography, (photoplethysmography, PPG)) is mounted on the bottom housing, and the mainboard is mounted on the middle frame. With development of electronic technologies, users have increasingly high requirements on appearances and performance of electronic devices. How to implement miniaturization of the electronic device is an urgent problem to be resolved currently.

### SUMMARY

Embodiments of this application provide a circuit board assembly and an electronic device, to implement miniaturization of an electronic device having a detection module.

The following technical solutions are used in embodiments of this application.

According to a first aspect, an embodiment of this application provides a circuit board assembly, including a mainboard, an electromagnetic shielding member, and a detection module. The mainboard has a first surface and a second surface that face away from each other. The electromagnetic shielding member has a third surface and a fourth surface that face away from each other, the electromagnetic shielding member is disposed on the first surface of the mainboard, and the third surface of the electromagnetic shielding member and the first surface of the mainboard are disposed facing each other. The detection module includes a substrate and a detection unit, the substrate has a fifth surface and a sixth surface that face away from each other, the fifth surface of the substrate is fastened to the fourth surface of the electromagnetic shielding member, the detection unit is mounted on the sixth surface of the substrate, and the substrate is electrically connected to the mainboard.

According to the circuit board assembly provided in this embodiment of this application, the electromagnetic shielding member is disposed on the mainboard, the detection module may be disposed on the electromagnetic shielding member as an independent module, the detection module is electrically connected to the mainboard, and the mainboard, the electromagnetic shielding member, and the detection module are stacked and assembled to form the circuit board assembly. When being used in an electronic device as an integral assembly, the circuit board assembly may be directly assembled on a middle frame of the electronic device. A safety gap may not be reserved between the electromagnetic shielding member and the detection module, and a support with a specific thickness may not be disposed on a bottom housing. The circuit board assembly in this embodiment can be miniaturized, and an overall thickness is small, allowing the entire device to be thinned by more than 0.15 mm. When being dropped or squeezed in a production process or a user use scenario, the detection module, the electromagnetic shielding member, and the mainboard undergo synchronous micro-deformation, to reduce a failure or damage caused by collision of the detection module.

In an optional implementation, the fifth surface of the substrate may be bonded through adhesive, welded, riveted, snap-fitted, or connected through a fastener (such as a screw) to the fourth surface of the electromagnetic shielding member. This facilitates implementation of a reliable connection between the substrate and the electromagnetic shielding member.

In an optional implementation, when the substrate is connected to the electromagnetic shielding member through the adhesive, the adhesive may be a double-sided adhesive. The double-sided adhesive is disposed on the substrate or the electromagnetic shielding member, and then the substrate and the electromagnetic shielding member are bonded through the double-sided adhesive. The double-sided adhesive is an adhesive layer disposed between the substrate and the electromagnetic shielding member.

In an optional implementation, when the substrate is connected to the electromagnetic shielding member through the adhesive, the adhesive may be dispensed glue. A liquid adhesive is disposed on the substrate or the electromagnetic shielding member, and after the substrate and the electromagnetic shielding member are pressed together, and the liquid adhesive is cured, the substrate is connected to the electromagnetic shielding member.

In an optional implementation, when the substrate is connected to the electromagnetic shielding member through a snap fit, the substrate has a snap fit slot, and the electromagnetic shielding member has the snap fit. The snap fit is snap-fitted into the snap fit slot, so that the substrate can be fastened to the electromagnetic shielding member.

In an optional implementation, a first electronic element is disposed on the first surface of the mainboard, and the electromagnetic shielding member covers the first electronic element. The electromagnetic shielding member includes a plate-shaped portion and a flanged portion, the flanged portion is connected to an edge of the plate-shaped portion, the flanged portion is fastened to the first surface of the mainboard, an end face that is of the plate-shaped portion and that faces the mainboard forms the third surface, and an end face that is of the plate-shaped portion and that faces away from the mainboard forms the fourth surface. The plate-shaped portion is configured to implement electromagnetic shielding of the first electronic element, and is further used as a supporting part of the substrate in the detection module. The flanged portion may be connected to the first surface of the mainboard, so that the electromagnetic shielding member is fastened to the mainboard.

In an optional implementation, the substrate may have a first flexible extension segment, and the first flexible extension segment is electrically connected to the mainboard. The mainboard, the electromagnetic shielding member, and the detection module are assembled to form an integral structure of the circuit board assembly, and the first flexible extension segment with a short length and a small area is configured between the mainboard and the substrate, so that the mainboard is electrically connected to the detection module, and costs are low.

In an optional implementation, a connection position between the first flexible extension segment and the mainboard is located on the first surface of the mainboard, and is disposed close to an edge of the electromagnetic shielding member. The mainboard is electrically connected to the substrate through the first flexible extension segment, so that the length of the first flexible extension segment is short, and the area of the first flexible extension segment is small, thereby effectively reducing costs.

In an optional implementation, the substrate has one or more second flexible extension segments, and a second electronic element is disposed on each of the one or more second flexible extension segments.

The second electronic element having a predetermined function may be mounted on the second flexible extension segment, and a position and a posture of the second flexible extension segment are adjusted, so that the second electronic element is adjusted to a predetermined position. The substrate is configured with a plurality of second flexible extension segments according to requirements, and is configured with second electronic elements with different functions, to implement different function extensions.

In an optional implementation, the substrate includes a flexible circuit board and a reinforcement sheet. The flexible circuit board and the reinforcement sheet are stacked. The first flexible extension segment is formed on the flexible circuit board, the reinforcement sheet is not disposed on the first flexible extension segment, and the detection unit is disposed in a region that is of the flexible circuit board and that corresponds to the reinforcement sheet, and the reinforcement sheet is fastened to the fourth surface of the electromagnetic shielding member.

The flexible circuit board is used as a carrier for mounting the detection unit, the first flexible extension segment is used as a flexible extension part for implementing the connection between the substrate and the mainboard, and the reinforcement sheet can increase structural strength. The reinforcement sheet is fastened to the electromagnetic shielding member, so that the detection module is reliably fastened to the electromagnetic shielding member.

In an optional implementation, the substrate includes a first rigid board and a first flexible board, the first rigid board is electrically connected to the first flexible board, the first flexible board is used as the first flexible extension segment, the detection unit is disposed on the first rigid board, and the first rigid board is fastened to the fourth surface of the electromagnetic shielding member.

The first rigid board is used as a carrier for mounting the detection unit, and the first flexible board is used as a flexible extension part for implementing a connection between the first rigid board and the mainboard. The first rigid board is fastened to the electromagnetic shielding member, so that the detection module is reliably fastened to the electromagnetic shielding member.

In an optional implementation, the substrate is a rigid-flexible board, the rigid-flexible board includes a first rigid portion and a first flexible portion, the first rigid portion is electrically connected to the first flexible portion, the first flexible portion is used as the first flexible extension segment, the detection unit is disposed on the first rigid portion, and the first rigid portion is fastened to the fourth surface of the electromagnetic shielding member.

The first rigid portion and the first flexible portion are of an integrated structure, the first rigid portion is used as a carrier for mounting the detection unit, and the first flexible portion is used as a flexible extension part for implementing a connection between the first rigid portion and the mainboard. The first rigid portion is fastened to the electromagnetic shielding member, so that the detection module is reliably fastened to the electromagnetic shielding member.

In an optional implementation, the substrate and the mainboard are electrically connected through a connector, the connector includes a first connecting portion and a second connecting portion that are plug-connected and fit with each other, the first connecting portion is disposed on the substrate, and the second connecting portion is disposed on the mainboard. The substrate and the mainboard are connected through the connector, so that assembling is easy, and the substrate is electrically connected to the mainboard.

In an optional implementation, the connector may be a board-to-board connector, a zero insertion force connector, or a connector of another type.

In an optional implementation, when the substrate has the first flexible extension segment, the first connecting portion may be disposed on the first flexible extension segment. After the substrate is fastened to the electromagnetic shielding member, the position of the first flexible extension segment is adjusted, so that the first connecting portion and the second connecting portion are plug-connected and fit with each other. In this way, assembling is convenient and reliable.

In an optional implementation, when the first flexible extension segment is not disposed on the substrate, the first connecting portion may be disposed on a side that is of the substrate and that faces the mainboard, and the second connecting portion may be disposed on a side that is of the mainboard and that faces the substrate. The first connecting portion and the second connecting portion are plug-connected and fit with each other, so that the substrate is electrically connected to the mainboard.

In an optional implementation, the substrate is electrically connected to the mainboard through welding, the substrate has a first pad, the mainboard has a second pad, and the first pad is welded to the second pad. The substrate and the mainboard are connected through the pads, so that assembling is easy, and the substrate is electrically connected to the mainboard.

In an optional implementation, the detection unit may include at least one of a photoplethysmography sensor, an electrocardiogram sensor, and a temperature sensor. In other words, the detection unit with one or more functions may be configured according to requirements. Different detection units may monitor different physiological parameters of the user.

In an optional implementation, when the detection unit includes the photoplethysmography sensor, the photoplethysmography sensor includes one or more light emitting diodes and one or more photoelectric diodes, a light emitting surface of the light emitting diode is disposed facing away from the mainboard, and a light receiving surface of the photoelectric diode is disposed facing away from the mainboard.

In an optional implementation, a light shielding member is disposed between the light emitting diode and the photoelectric diode. This prevents the light of the light emitting diode from directly irradiating on the photoelectric diode, so that the photoelectric diode receives the optical signal reflected by the human body, thereby improving detection accuracy.

In an optional implementation, the light shielding member may be made of a material such as foam. The light shielding member may be set to be in a shape of a rectangle, a circle, or the like, and is disposed around the light emitting diode. An area of the light shielding member is set according to requirements. The light shielding member may be fastened to the substrate in a manner of bonding or the like.

In an optional implementation, an inner wall of the bottom housing may have a separation rib. In a process in which the bottom housing is fastened to the middle frame, the separation rib abuts against the light shielding member, to implement light shielding effect between the light emitting diode and the photoelectric diode.

In an optional implementation, two photoelectric diodes, two photoelectric diodes, and one frame-shaped light shielding member are disposed on the substrate. The photoelectric diodes and the photoelectric diodes are arranged in a staggered manner in a predetermined direction, and the light shielding member is disposed between the two adjacent photoelectric diodes and is disposed around one photoelectric diode.

In an optional implementation, a first lens is disposed in front of the light emitting surface of the light emitting diode, and a second lens is disposed in front of the light receiving surface of the photoelectric diode. The light emitting diode is combined with the first lens, so that light generated by the light emitting diode is well emitted. The photoelectric diode is combined with the second lens, so that external light is well received by the photoelectric diode.

In an optional implementation, a battery is fastened to the second surface of the mainboard, and the battery is electrically connected to the mainboard. The battery may be configured to supply power to components such as the mainboard and the detection module. In this way, a size and a capacity of the battery can be large, increasing maximum operating time of the entire device. The battery may be disposed in the middle frame.

According to a second aspect, an embodiment of this application provides an electronic device. The electronic device includes the foregoing circuit board assembly, a bottom housing, and a middle frame, the bottom housing is connected to the middle frame, the circuit board assembly includes the mainboard, the electromagnetic shielding member, and the detection module, the mainboard has the first surface and the second surface that face away from each other, the mainboard is disposed on the middle frame, the first surface of the mainboard and the bottom housing are disposed facing each other, the electromagnetic shielding member is disposed on the first surface of the mainboard, the detection module is disposed on the electromagnetic shielding member, the detection module is electrically connected to the mainboard, and the detection module is disposed close to the bottom housing.

According to the electronic device provided in this embodiment of this application, the electromagnetic shielding member is disposed on the mainboard, the detection module may be disposed on the electromagnetic shielding member as an independent module, the mainboard, the electromagnetic shielding member, and the detection module are stacked and assembled to form the circuit board assembly, the circuit board assembly is assembled on the middle frame, and the bottom housing is mounted on the middle frame. The detection module is electrically connected to the mainboard to implement signal transmission. A safety gap may not be reserved between the electromagnetic shielding member and the detection module, and a support with a specific thickness may not be disposed on the bottom housing. The electronic device in this embodiment can be miniaturized, and an overall thickness is small, allowing the entire device to be thinned by more than 0.15 mm. When being dropped or squeezed in a production process or a user use scenario, the detection module, the electromagnetic shielding member, and the mainboard undergo synchronous micro-deformation, to reduce a failure or damage caused by collision of the detection module. The electronic device in this embodiment is easy to assemble, and an assembly process is simple. There is no need to use a heat stake on the bottom housing to fasten the detection module, and a Mylar sheet does not need to be attached to an outer surface of the heat stake.

In an optional implementation, the electronic device is a watch, a band, or another wearable device, the bottom housing is mounted on the middle frame, and the middle frame may be connected to a watchband or a wristband, to facilitate wearing and using by a user. When a user wears a wearable device, the detection module is disposed close to the bottom housing, the detection module faces skin of the user, and information such as a physiological indicator of the user may be detected through the detection module.

In an optional implementation, the mainboard and the middle frame may be connected in different manners, for example, may be connected through a fastener (such as a screw), a snap fit, riveting, bonding, or the like, so that the circuit board assembly is assembled as a whole on the middle frame.

In an optional implementation, the mainboard has a plurality of through holes, and the middle frame has a plurality of threaded holes. The through hole of the mainboard is aligned with the threaded hole of the middle frame, and the fastener passes through the through hole and is in a threaded connection to the threaded hole, so that the circuit board assembly can be reliably fastened to the middle frame.

In an optional implementation, the mainboard has a snap fit slot, and the middle frame has a snap fit. The snap fit is snap-fitted into the snap fit slot, so that the circuit board assembly can be reliably fastened to the middle frame.

In an optional implementation, the middle frame and the bottom housing may be connected through adhesive bonding or a snap fit, or in another manner, to implement a reliable connection between the middle frame and the bottom housing.

In an optional implementation, a screen assembly is further included, the screen assembly is disposed on the middle frame, the screen assembly is electrically connected to the mainboard, and the second surface of the mainboard and the screen assembly are disposed facing each other. The screen assembly is configured to output light to display information such as an image and a text. The screen assembly and the bottom housing may be respectively disposed at two opposite ends of the middle frame. The screen assembly presents information to the user, and the detection module close to the bottom housing performs detection on the user.

In an optional implementation, there may be a touch layer on the screen assembly, and the touch layer is electrically connected to the mainboard. The touch layer is configured to detect a touch operation performed on or near the touch layer.

### BRIEF DESCRIPTION OF DRAWINGS

FIG. 1 is a sectional view of an electronic device in a related technology;
FIG. 2 is a partial enlarged view at a position A in FIG. 1;
FIG. 3 is a diagram of assembly of a detection module and a bottom housing in a related technology;
FIG. 4 is a diagram of a three-dimensional structure of the detection module in FIG. 3;
FIG. 5 is a sectional view of another electronic device in a related technology;
FIG. 6 is a three-dimensional exploded view of an electronic device according to an embodiment of this application;
FIG. 7 is a diagram of assembly of a part of a structure of the electronic device in FIG. 6;
FIG. 8 is a three-dimensional diagram of assembly of a circuit board assembly in the electronic device in FIG. 6;
FIG. 9 is a front view of the circuit board assembly in FIG. 8;
FIG. 10 is a side view of the circuit board assembly in FIG. 9;
FIG. 11 is a sectional view of the circuit board assembly in FIG. 8 along a line B-B;
FIG. 12 is a diagram of a structure of a detection module in the circuit board assembly in FIG. 9;
FIG. 13 is a diagram of a structure of a detection module in a circuit board assembly according to another embodiment of this application;
FIG. 14 is a diagram of a structure of a detection module in a circuit board assembly according to another embodiment of this application;
FIG. 15 is a sectional view of an electronic device according to an embodiment of this application; and
FIG. 16 is a partial enlarged view at a position C in FIG. 15.

### Reference numerals:

10: electronic device; 11: middle frame; 12: bottom housing; 13: mainboard; 13a: electronic element; 14: electromagnetic shielding member; 15: detection module; 15a: substrate; 15b: flexible printed circuit; 16: battery; 17: gap; 18: heat stake;
20: electronic device; 21: middle frame; 22: bottom housing; 23: mainboard; 24: sub-board; 25: detection module; 25a: substrate; 25b: flexible printed circuit; 26: battery; 27: support; 28: gap; 29: flexible circuit board;
100: circuit board assembly;
110: mainboard; 110a: first surface; 110b: second surface; 111: through hole; 112: first electronic element;
120: electromagnetic shielding member; 120a: third surface; 120b: fourth surface; 121: plate-shaped portion; 122: flanged portion; 123: edge;
130: detection module; 131: substrate; 131a: fifth surface; 131b: sixth surface; 131c: flexible circuit board; 131d: reinforcement sheet; 131e: first rigid board; 131f: first flexible board; 131g: connector; 131h: first rigid portion; 131i: first flexible portion; 1311: first flexible extension segment; 1311a: connection position; 1312: second flexible extension segment; 132: detection unit; 1321: light emitting diode; 1322: photoelectric diode; 1323: light shielding member; 1324: first lens; 1325: second lens; 133: predetermined component;
140: adhesive; 150: second electronic element; 160: connector; 161: first connecting portion; 162: second connecting portion; 170: battery;
200: bottom housing; 201: separation rib; 300: middle frame; 301: fastener; 400: screen assembly; 1000: electronic device.

### DESCRIPTION OF EMBODIMENTS

To make technical problems to be resolved, technical solutions, and beneficial effects of this application clearer, the following further describes this application in detail with reference to accompanying drawings and embodiments. It should be understood that the specific embodiments described herein are merely used to explain this application but are not intended to limit this application. Although this application is described with reference to some embodiments, it does not mean that a characteristic of this application is limited only to this implementation. On the contrary, a purpose of describing this application with reference to an implementation is to cover another option or modification that may be derived based on claims of this application. To provide an in-depth understanding of this application, the following descriptions include a plurality of specific details. This application may be alternatively implemented without using these details. In addition, to avoid confusion or blurring a focus of this application, some specific details are omitted from the description. It should be noted that embodiments in this application and the features in embodiments may be mutually combined in the case of no conflict.

It should be noted that when an element is referred to as "fastened to" or "disposed on" another element, the element may be directly or indirectly on the another element. When an element is referred to as "connected to" another element, the element may be directly or indirectly connected to the another element.

It should be understood that in descriptions of embodiments of this application, it should be noted that, unless otherwise clearly specified and limited, terms "mounting" and "connection" should be understood in a broad sense. For example, "connection" may be a detachable connection, a nondetachable connection, a direct connection, or an indirect connection through an intermediate medium. Orientation or position relationships indicated by terms "length", "width", "upper", "lower", "front", "rear", "left", "right", "vertical", "horizontal", "top", "bottom", "inner", "outer", and the like are orientation or position relationships based on the accompanying drawings. This is merely intended to facilitate and simplify the descriptions of this application, but is not intended to indicate or imply that an indicated apparatus or element needs to have a specific orientation or be constructed and operated in a specific orientation. Therefore, such terms cannot be construed as a limitation on this application.

In addition, the terms "first" and "second" are merely intended for a purpose of description, and shall not be understood as an indication or implication of relative importance or implicit indication of a quantity of indicated technical features. Therefore, a feature limited by "first" or "second" may explicitly or implicitly include one or more features. In the descriptions of this application, "a plurality of" means two or more, unless otherwise explicitly and specifically defined.

Reference to "an embodiment", "some embodiments", or the like described in this specification means that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "have", and their variants all mean "include but are not limited to", unless otherwise specifically emphasized in another manner.

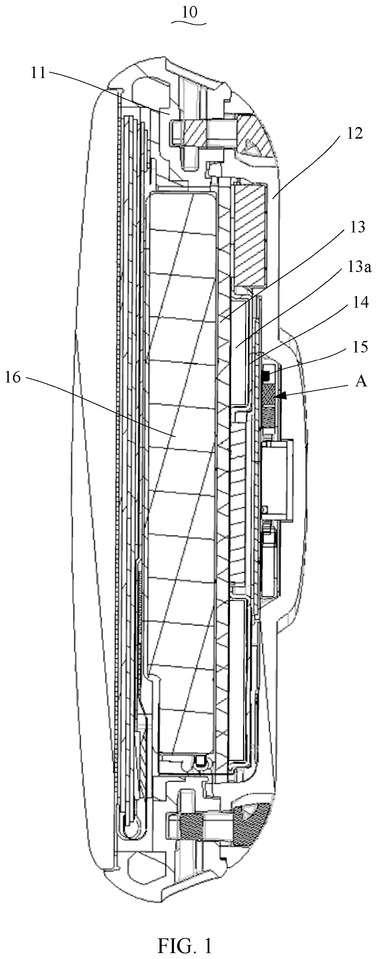

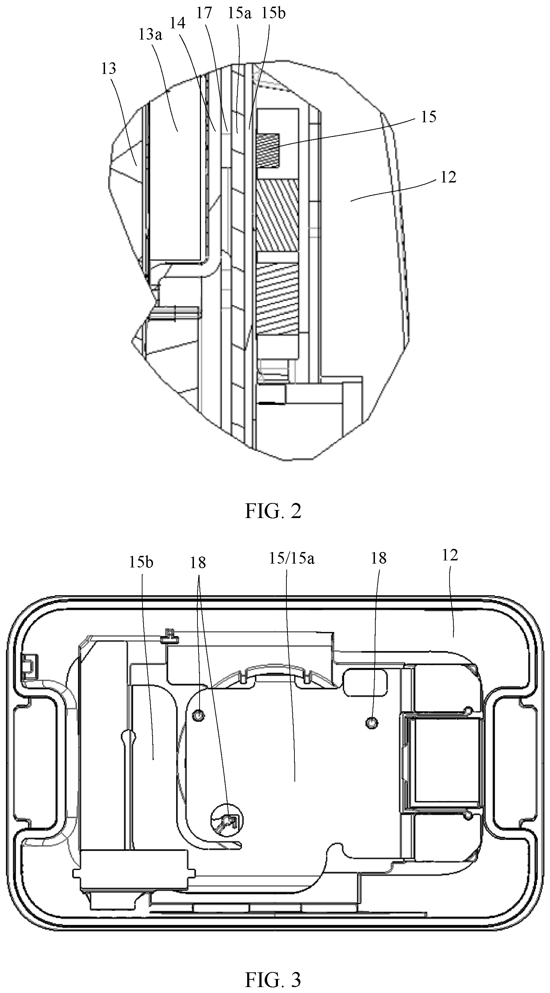

FIG. 1 and FIG. 2 show an electronic device 10 in a related technology. The electronic device 10 may include a middle frame 11, a bottom housing 12, a mainboard 13, an electromagnetic shielding member 14, a detection module 15, and a battery 16. The detection module 15 is mounted on the bottom housing 12, the mainboard 13 is mounted on the middle frame 11, the battery 16 is assembled on a top surface of the mainboard 13, and an electronic element 13a such as a communication chip may be mounted on a bottom surface of the mainboard 13. The electromagnetic shielding member 14 is disposed on the bottom surface of the mainboard 13 and covers the electronic element 13a. The bottom surface of the mainboard 13 and the bottom housing 12 are disposed facing each other, and the bottom housing 12 is mounted on the middle frame 11. A safety gap 17 greater than or equal to 0.15 millimeters (mm) needs to be reserved between the electromagnetic shielding member 14 and a substrate 15a in the detection module 15, to reduce a failure or damage caused by collision of the detection module 15 when the detection module 15 undergoes instantaneous micro-deformation due to a drop or squeeze in a production process or a user use scenario. The safety gap 17 increases an overall thickness of the electronic device 10 (a size in a left-right direction in FIG. 1 and FIG. 2).

With reference to FIG. 3, when the detection module 15 is mounted on the bottom housing 12, the substrate 15a of the detection module 15 needs to pass through a heat stake 18 of the bottom housing 12, and the substrate 15a is fastened to the bottom housing 12 through heat staking, so that the detection module 15 is fastened to the bottom housing 12. A Mylar sheet may be attached to the outer surface of the heat stake 18, to address stringing and roughness issues on the outer surface of the heat stake 18. These complicate a manufacturing process.

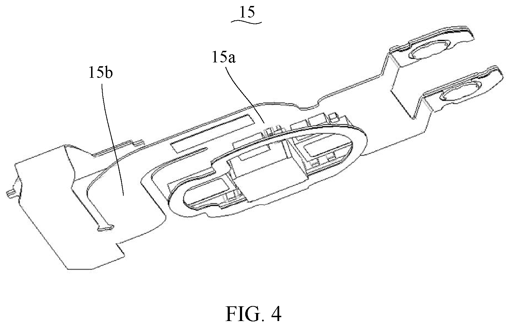

With reference to FIG. 3 and FIG. 4, the detection module 15 is mounted on the bottom housing 12, and the substrate 15a has a redundant flexible printed circuit (flexible printed circuit, FPC) 15b. Before the bottom housing 12 is mounted on the middle frame 11, the FPC 15b of the detection module 15 needs to be connected to the mainboard 13 through a connector, to implement a conductive connection between the mainboard 13 and the detection module 15. The FPC 15b of the detection module 15 needs to be designed with reference to a gap between the electromagnetic shielding member 14 and the bottom housing 12 and a specific path. As a result, the FPC 15b has a long length and a large area, and costs are high.

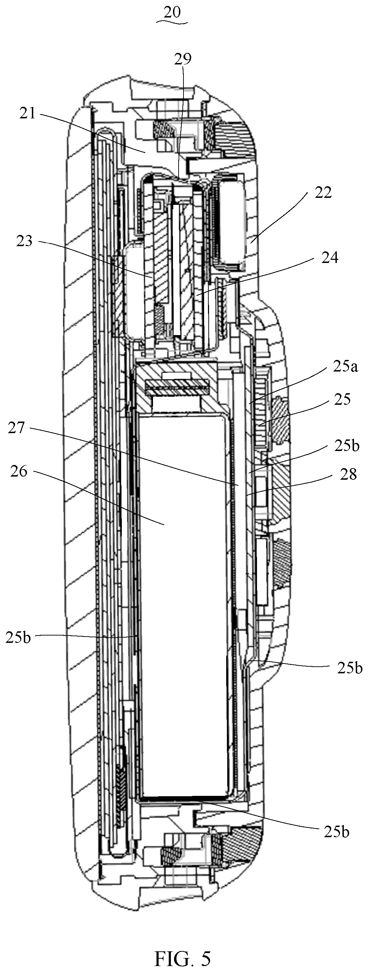

FIG. 5 shows another electronic device 20 in a related technology. The electronic device 20 may include a middle frame 21, a bottom housing 22, a mainboard 23, a sub-board 24, a detection module 25, a battery 26, and a support 27. The detection module 25 is mounted on the bottom housing 22, a thickness of the support 27 is approximately 0.6 mm, the support 27 is mounted on the bottom housing 22 and is located above the detection module 25 (on a left side of the detection module 25 in FIG. 5), and the battery 26 is mounted on the support 27. The mainboard 23 and the sub-board 24 are mounted on a side of the battery 26, that is, beside the battery 26 in a direction perpendicular to a thickness direction of the battery 26 (the thickness direction of the battery in FIG. 5 is a left-right direction). A safety gap 28 greater than or equal to 0.15 mm needs to be reserved between the support 27 and a substrate 25a in the detection module 25, to reduce a failure or damage caused by collision of the detection module 25 when the detection module 25 undergoes instantaneous micro-deformation due to a drop or squeeze in a production process or a user use scenario. The thickness of the safety gap 28 and the thickness of the support 27 increase an overall thickness of the electronic device 20 (a size in a left-right direction in FIG. 5).

The mainboard 23 and the sub-board 24 are disposed on the side of the battery 26. As a result, a size and a capacity of the battery 26 are small, affecting maximum operating time of the entire device. The mainboard 23 and the sub-board 24 need to be connected through a flexible circuit board 29, which complicates a manufacturing process.

The detection module 25 is mounted on the bottom housing 22, and the substrate 25a has a redundant flexible printed circuit 25b. Before the bottom housing 22 is mounted on the middle frame 21, the FPC 25b of the detection module 25 needs to be connected to the mainboard 23 through a connector, to implement a conductive connection between the mainboard 23 and the detection module 25. The FPC 25b of the detection module 25 needs to be designed with reference to a gap between a plurality of components and a specific path, and the FPC 25b needs to extend along a bottom, a side, and a top of the battery 26. As a result, the FPC 25b has a long length and a large area, and costs are high.

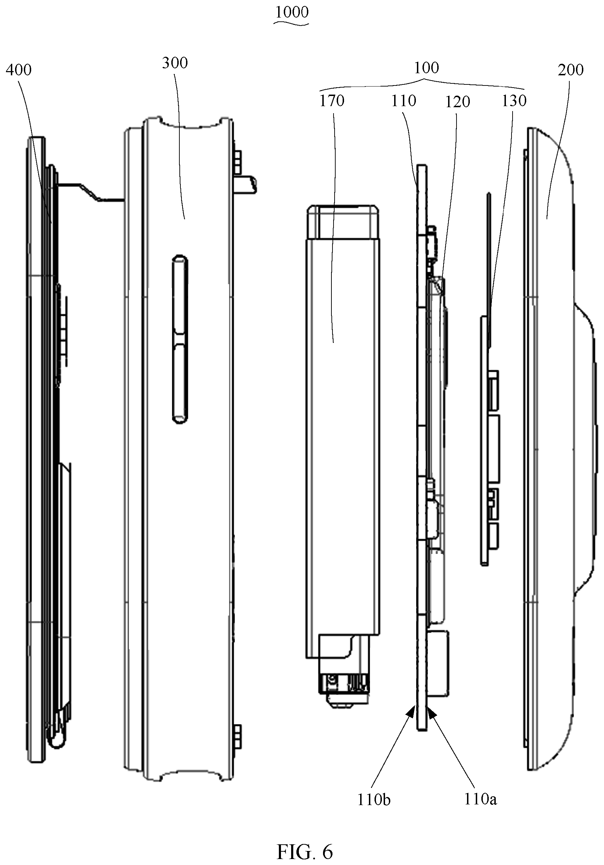

Refer to FIG. 6 to FIG. 8. An embodiment of this application provides an electronic device 1000, including a circuit board assembly 100, a bottom housing 200, and a middle frame 300. The bottom housing 200 is connected to the middle frame 300. The circuit board assembly 100 includes a mainboard 110, an electromagnetic shielding member 120, and a detection module 130. The mainboard 110 has a first surface 110a and a second surface 110b that face away from each other. The mainboard 110 is disposed on the middle frame 300, and the first surface 110a of the mainboard 110 and the bottom housing 200 are disposed facing each other. The electromagnetic shielding member 120 is disposed on the first surface 110a of the mainboard 110, the detection module 130 is disposed on the electromagnetic shielding member 120, the detection module 130 is electrically connected to the mainboard 110, and the detection module 130 is disposed close to the bottom housing 200.

The electronic device 1000 may be various devices having the detection module 130, for example, a watch, a band, or another wearable device. The bottom housing 200 may be disposed on one side of the middle frame 300, and both the bottom housing 200 and the middle frame 300 are used as housing parts of the electronic device 1000. Structures such as the circuit board assembly 100 and a battery 170 may be disposed in the middle frame 300.

Electronic elements such as a processor and a communication chip may be disposed on the mainboard 110. The electromagnetic shielding member 120 may cover some electronic elements on the mainboard 110, to implement electromagnetic shielding between the electronic elements. The electromagnetic shielding member 120 may support the detection module 130. The detection module 130 is electrically connected to the mainboard 110 to implement signal transmission.

For example, the electronic device 1000 is a watch, a band, or another wearable device, the bottom housing 200 is mounted on the middle frame 300, and the middle frame 300 may be connected to a watchband or a wristband, to facilitate wearing and using by a user. When the user wears the wearable device, the detection module 130 is disposed close to the bottom housing 200, the detection module 130 faces skin of the user, and information such as a physiological indicator of the user may be detected through the detection module 130.

According to the electronic device 1000 provided in this embodiment of this application, the electromagnetic shielding member 120 is disposed on the mainboard 110, the detection module 130 may be disposed on the electromagnetic shielding member 120 as an independent module, the mainboard 110, the electromagnetic shielding member 120, and the detection module 130 are stacked and assembled to form the circuit board assembly 100, the circuit board assembly 100 is assembled on the middle frame 300, and the bottom housing 200 is mounted on the middle frame 300. The detection module 130 is electrically connected to the mainboard 110 to implement signal transmission. A safety gap may not be reserved between the electromagnetic shielding member 120 and the detection module 130, and a support with a specific thickness may not be disposed on the bottom housing 200. The electronic device 1000 in this embodiment can be miniaturized, and an overall thickness is small, allowing the entire device to be thinned by more than 0.15 mm. When being dropped or squeezed in a production process or a user use scenario, the detection module 130, the electromagnetic shielding member 120, and the mainboard 110 undergo synchronous micro-deformation, to reduce a failure or damage caused by collision of the detection module 130. The electronic device 1000 in this embodiment is easy to assemble, and an assembly process is simple. There is no need to use a heat stake on the bottom housing to fasten the detection module, and a Mylar sheet does not need to be attached to an outer surface of the heat stake.

When the mainboard 110 of the circuit board assembly 100 and the middle frame 300 are assembled, as shown in FIG. 6 to FIG. 8, the mainboard 110 and the middle frame 300 may be connected in different manners, for example, may be connected through a fastener 301 (such as a screw), a snap fit, riveting, bonding, or the like, so that the circuit board assembly 100 is assembled as a whole on the middle frame 300.

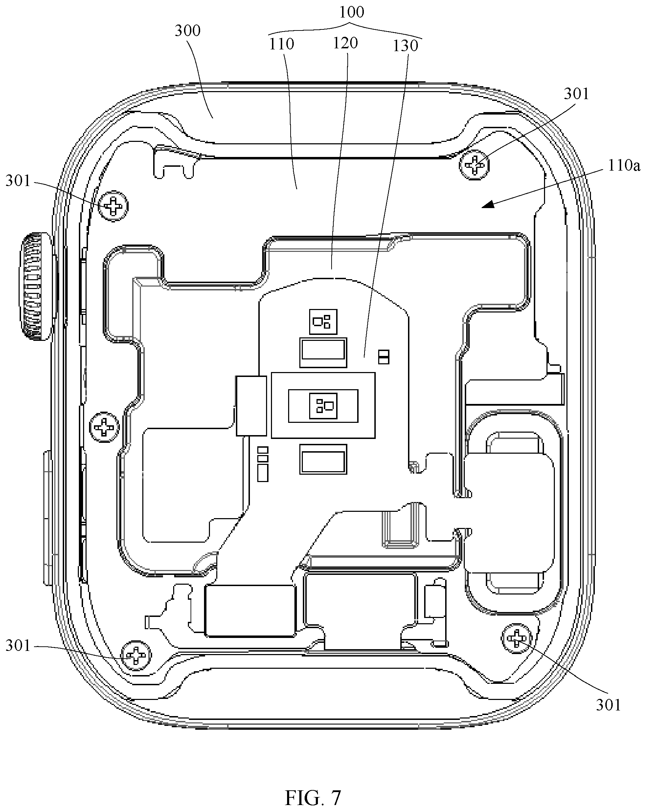

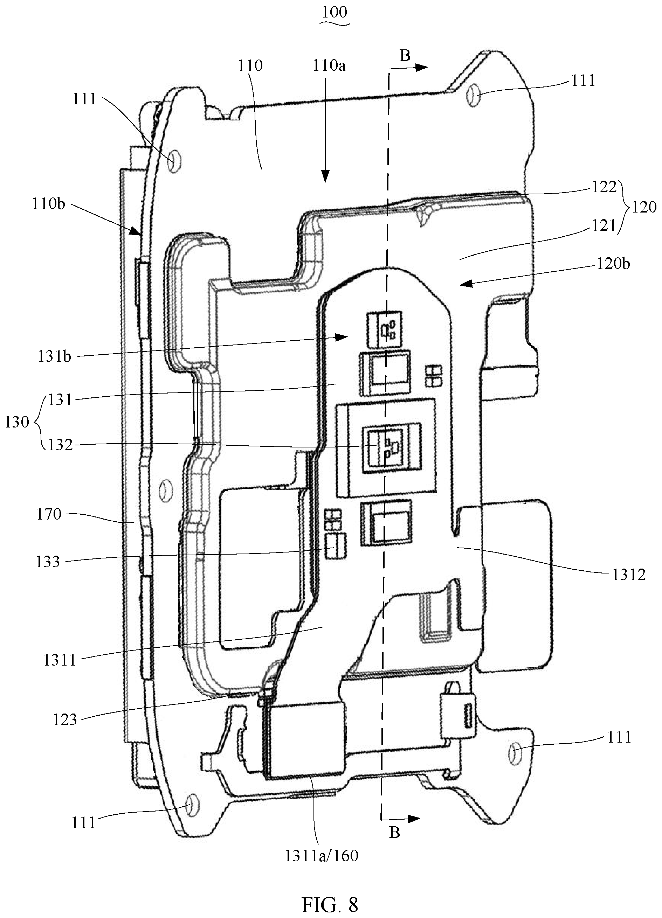

For example, as shown in FIG. 7 and FIG. 8, the mainboard 110 has a plurality of through holes 111, and the middle frame 300 has a plurality of threaded holes (not shown in the figure). The through hole 111 of the mainboard 110 is aligned with the threaded hole of the middle frame 300, and the fastener 301 passes through the through hole 111 and is in a threaded connection to the threaded hole, so that the circuit board assembly 100 can be reliably fastened to the middle frame 300.

For example, the mainboard 110 has a snap fit slot (not shown in the figure), and the middle frame 300 has a snap fit (not shown in the figure). The snap fit is snap-fitted into the snap fit slot, so that the circuit board assembly 100 can be reliably fastened to the middle frame 300.

When the middle frame 300 and the bottom housing 200 are assembled, as shown in FIG. 5, the middle frame 300 and the bottom housing 200 may be connected through adhesive bonding or a snap fit, or in another manner, to implement a reliable connection between the middle frame 300 and the bottom housing 200.

In some embodiments, as shown in FIG. 6, a screen assembly 400 is further included, the screen assembly 400 is disposed on the middle frame 300, the screen assembly 400 is electrically connected to the mainboard 110, and the second surface 110b of the mainboard 110 and the screen assembly 400 are disposed facing each other, so that the electronic device 1000 has a display function.

The screen assembly 400 is electrically connected to the mainboard 110 to implement signal transmission. The screen assembly 400 is configured to output light to display information such as an image and a text, for example, the physiological indicator, of the user, detected by the detection module 130. The screen assembly 400 and the bottom housing 200 may be respectively disposed at two opposite ends of the middle frame 300. The screen assembly 400 presents information to the user, and the detection module 130 close to the bottom housing 200 performs detection on the user.

The screen assembly 400 may be an organic light emitting diode (organic light emitting diode, OLED) screen, an active-matrix organic light emitting diode (active-matrix organic light emitting diode, AMOLED) screen, a mini light emitting diode (Mini LED) screen, a micro light emitting diode (Micro LED) screen, a micro organic light emitting diode (Micro OLED) screen, a quantum dot light emitting diode (quantum dot light emitting diode, QLED) screen, or the like.

In some embodiments, there may be a touch layer (not shown in the figure) on the screen assembly 400, and the touch layer is electrically connected to the mainboard 110, so that the electronic device 1000 has a touch function. The touch layer is configured to detect a touch operation performed on or near the touch layer. The touch layer may transfer the detected touch operation to a processor, to determine a touch event type. A visual output related to the touch operation is provided through the screen assembly 400. The touch layer may be a conventional capacitive touch sensor or another type of touch sensor.

Refer to FIG. 8 to FIG. 11. An embodiment of this application provides a circuit board assembly 100, including the mainboard 110, the electromagnetic shielding member 120, and the detection module 130. The mainboard 110 has the first surface 110a and the second surface 110b that face away from each other. The electromagnetic shielding member 120 has a third surface 120a and a fourth surface 120b that face away from each other. The electromagnetic shielding member 120 is disposed on the first surface 110a of the mainboard 110, and the third surface 120a of the electromagnetic shielding member 120 and the first surface 110a of the mainboard 110 are disposed facing each other. The detection module 130 includes a substrate 131 and a detection unit 132. The substrate 131 has a fifth surface 131a and a sixth surface 131b that face away from each other. The fifth surface 131a of the substrate 131 is fastened to the fourth surface 120b of the electromagnetic shielding member 120. The detection unit 132 is mounted on the sixth surface 131b of the substrate 131. The substrate 131 is electrically connected to the mainboard 110.

An electronic element may be disposed on both the first surface 110a and the second surface 110b of the mainboard 110. The substrate 131 in the detection module 130 may be used as a carrier of the detection unit 132 or another predetermined component 133. The detection unit 132 is a hardware structure, and the detection unit 132 may have different representation forms.

For example, when the detection module 130 is a photoplethysmography module, the detection unit 132 may include a light emitting diode 1321 and a photoelectric diode 1322. Alternatively, when the detection module 130 is an electrocardiogram module, the detection unit 132 is an electrocardiogram sensor. Alternatively, when the detection module 130 is a temperature detection module, the detection unit 132 is a temperature sensor.

That the fifth surface 131a of the substrate 131 is fastened to the fourth surface 120b of the electromagnetic shielding member 120 means that the fifth surface 131a of the substrate 131 and the fourth surface 120b of the electromagnetic shielding member 120 are mechanically connected face to face, and there is no gap between the fifth surface 131a of the substrate 131 and the fourth surface 120b of the electromagnetic shielding member 120.

According to the circuit board assembly 100 provided in this embodiment of this application, the electromagnetic shielding member 120 is disposed on the mainboard 110, the detection module 130 may be disposed on the electromagnetic shielding member 120 as an independent module, the detection module 130 is electrically connected to the mainboard 110, and the mainboard 110, the electromagnetic shielding member 120, and the detection module 130 are stacked and assembled to form the circuit board assembly 100. When being used in the electronic device 1000 as an integral assembly, the circuit board assembly 100 may be directly assembled on the middle frame 300 of the electronic device 1000. A safety gap may not be reserved between the electromagnetic shielding member 120 and the detection module 130, and a support with a specific thickness may not be disposed on the bottom housing 200. The circuit board assembly 100 in this embodiment can be miniaturized, and an overall thickness is small, allowing the entire device to be thinned by more than 0.15 mm. When being dropped or squeezed in a production process or a user use scenario, the detection module 130, the electromagnetic shielding member 120, and the mainboard 110 undergo synchronous micro-deformation, to reduce a failure or damage caused by collision of the detection module 130.

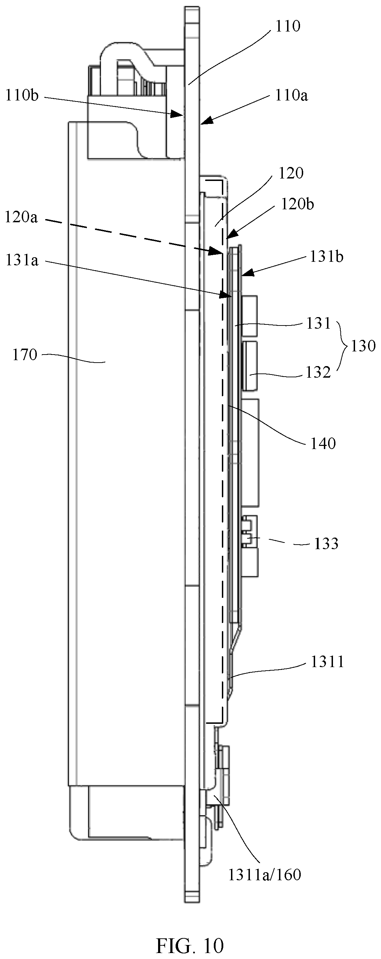

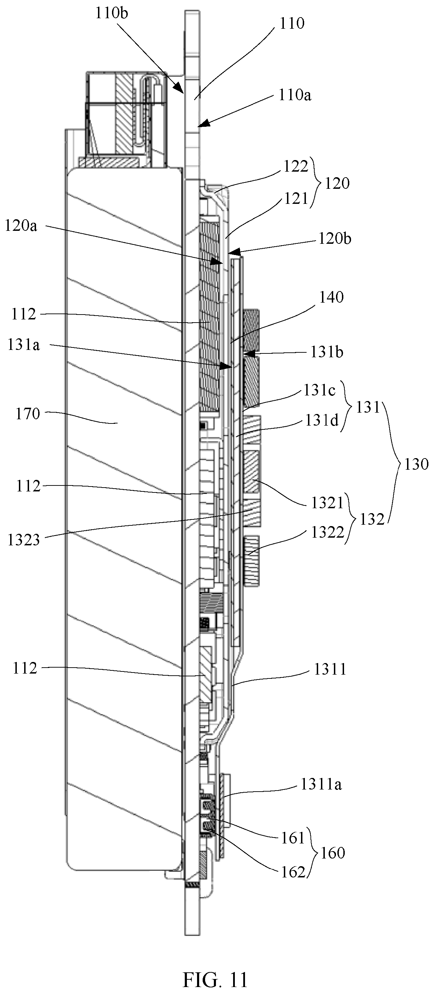

When the substrate 131 is connected to the electromagnetic shielding member 120, as shown in FIG. 10 and FIG. 11, the fifth surface 131a of the substrate 131 may be bonded through adhesive 140, welded, riveted, snap-fitted, or connected through a fastener (such as a screw) to the fourth surface 120b of the electromagnetic shielding member 120. The foregoing mechanical connection manner is used to facilitate implementation of a reliable connection between the substrate 131 and the electromagnetic shielding member 120.

For example, when the substrate 131 is connected to the electromagnetic shielding member 120 through the adhesive 140, the adhesive 140 may be a double-sided adhesive. The double-sided adhesive is disposed on the substrate 131 or the electromagnetic shielding member 120, and then the substrate 131 and the electromagnetic shielding member 120 are bonded through the double-sided adhesive. The double-sided adhesive is an adhesive layer disposed between the substrate 131 and the electromagnetic shielding member 120.

For example, when the substrate 131 is connected to the electromagnetic shielding member 120 through the adhesive 140, the adhesive 140 may be dispensed glue. A liquid adhesive is disposed on the substrate 131 or the electromagnetic shielding member 120, and after the substrate 131 and the electromagnetic shielding member 120 are pressed together, and the liquid adhesive is cured, the substrate 131 is connected to the electromagnetic shielding member 120.

For example, when the substrate 131 is connected to the electromagnetic shielding member 120 through a snap fit, the substrate 131 has a snap fit slot, and the electromagnetic shielding member 120 has the snap fit. The snap fit is snap-fitted into the snap fit slot, so that the substrate 131 can be fastened to the electromagnetic shielding member 120.

When the electromagnetic shielding member 120 is disposed, as shown in FIG. 11, a first electronic element 112 is disposed on the first surface 110a of the mainboard 110, and the electromagnetic shielding member 120 covers the first electronic element 112. The electromagnetic shielding member 120 includes a plate-shaped portion 121 and a flanged portion 122, the flanged portion 122 is connected to an edge of the plate-shaped portion 121, the flanged portion 122 is fastened to the first surface 110a of the mainboard 110, an end face that is of the plate-shaped portion 121 and that faces the mainboard 110 forms the third surface 120a, and an end face that is of the plate-shaped portion 121 and that faces away from the mainboard 110 forms the fourth surface 120b.

The electromagnetic shielding member 120 may cover the first electronic element 112 on the mainboard 110, to implement electromagnetic shielding of the first electronic element 112. The electromagnetic shielding member 120 may be made of metal such as iron or steel, and has good electromagnetic shielding effect. The first electronic element 112 may be a component such as a communication chip, a processor, or a memory. The plate-shaped portion 121 and the flanged portion 122 of the electromagnetic shielding member 120 may be of an integrated structure, for example, through stamping. The plate-shaped portion 121 is configured to implement electromagnetic shielding of the first electronic element 112, and is further used as a supporting part of the substrate 131 in the detection module 130. The flanged portion 122 may be connected to the first surface 110a of the mainboard 110, so that the electromagnetic shielding member 120 is fastened to the mainboard 110.

When the substrate 131 is electrically connected to the mainboard 110, in some embodiments, as shown in FIG. 8, FIG. 9, and FIG. 12, the substrate 131 may have a first flexible extension segment 1311, and the first flexible extension segment 1311 is electrically connected to the mainboard 110.

The first flexible extension segment 1311 is configured on the substrate 131, the first flexible extension segment 1311 may be understood as a bendable flexible circuit board, and a position and a posture of the first flexible extension segment 1311 are conveniently adjusted, so that the substrate 131 is electrically connected to the mainboard 110 to implement signal transmission. This embodiment is different from the technical solution shown in FIG. 3 and FIG. 4 in which the mainboard is disposed on the middle frame, the detection module is disposed on the bottom housing, and the redundant FPC is configured, to implement the electrical connection between the mainboard and the detection module in that the mainboard 110, the electromagnetic shielding member 120, and the detection module 130 are assembled to form an integral structure of the circuit board assembly 100, and the first flexible extension segment 1311 with a short length and a small area is configured between the mainboard 110 and the substrate 131, so that the mainboard 110 is electrically connected to the detection module 130, and costs are low.

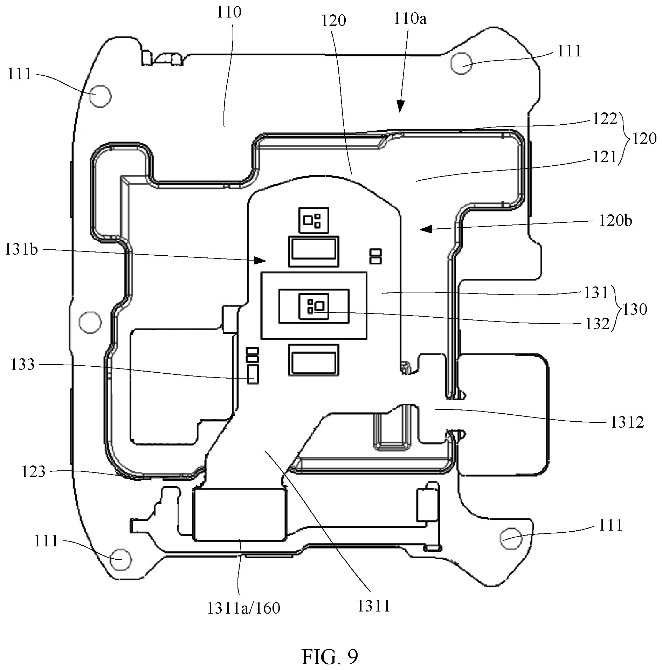

When a connection position 1311a between the first flexible extension segment 1311 and the mainboard 110 is disposed, as shown in FIG. 8 and FIG. 9, the connection position 1311a between the first flexible extension segment 1311 and the mainboard 110 is located on the first surface 110a of the mainboard 110, and is disposed close to an edge 123 of the electromagnetic shielding member 120. In this manner, the mainboard 110 is electrically connected to the substrate 131 through the first flexible extension segment 1311, so that the length of the first flexible extension segment 1311 is short, and the area of the first flexible extension segment 1311 is small, thereby effectively reducing costs.

For example, the first flexible extension segment 1311 with the small area may be disposed between the mainboard 110 and the detection module 130 in this embodiment, so that the mainboard 110 is electrically connected to the detection module 130. This embodiment is different from the technical solution shown in FIG. 3 and FIG. 4 in which the mainboard is disposed on the middle frame, the detection module is disposed on the bottom housing, and the mainboard and the detection module are electrically connected through the redundant FPC in that the area of the first flexible extension segment 1311 in this embodiment is reduced by more than 50%.

To enable the circuit board assembly 100 to have a specific function extension, in some embodiments, as shown in FIG. 8, FIG. 9, and FIG. 12, the substrate 131 has one or more second flexible extension segments 1312, and a second electronic element 150 is disposed on each of the one or more second flexible extension segments 1312.

The second flexible extension segment 1312 is configured on the substrate 131, and the second flexible extension segment 1312 may be understood as a bendable flexible circuit board. The second electronic element 150 having a predetermined function may be mounted on the second flexible extension segment 1312, and a position and a posture of the second flexible extension segment 1312 are adjusted, so that the second electronic element 150 is adjusted to a predetermined position. The second electronic element 150 is electrically connected to the mainboard 110 through the second flexible extension segment 1312 and the first flexible extension segment 1311, to implement driving control on the second electronic element 150. The second electronic element 150 may be a speaker, a vibration motor, or the like. The substrate 131 is configured with a plurality of second flexible extension segments 1312 according to requirements, and is configured with second electronic elements 150 with different functions, to implement different function extensions. The second electronic element 150 and the second flexible extension segment 1312 may be welded through a pad.

There are a plurality of optional implementations for manufacturing the substrate 131. The following provides three implementations as examples.

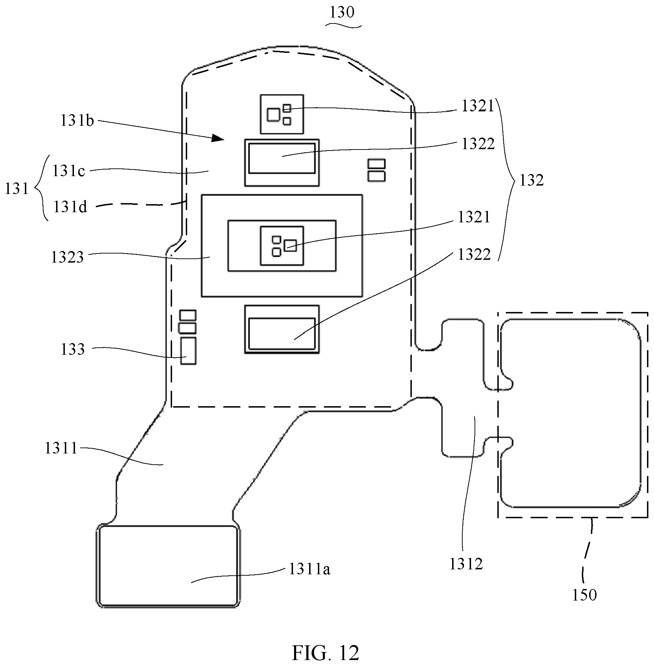

A first implementation of the substrate 131 is as follows: Refer to FIG. 11 and FIG. 12. The substrate 131 includes a flexible circuit board 131c and a reinforcement sheet 131d. The flexible circuit board 131c and the reinforcement sheet 131d are stacked. The first flexible extension segment 1311 is formed on the flexible circuit board 131c, the reinforcement sheet 131d is not disposed on the first flexible extension segment 1311, and the detection unit 132 is disposed in a region that is of the flexible circuit board 131c and that corresponds to the reinforcement sheet 131d, and the reinforcement sheet 131d is fastened to the fourth surface 120b of the electromagnetic shielding member 120.

The reinforcement sheet 131d is not disposed on the first flexible extension segment 1311, so that the first flexible extension segment 1311 is bendable, to facilitate adjusting the position and the posture of the first flexible extension segment 1311, thereby implementing the electrical connection between the first flexible extension segment 1311 and the mainboard 110. The region that is of the flexible circuit board 131c and that corresponds to the reinforcement sheet 131d is a region that is on the flexible circuit board 131c and that is connected to the reinforcement sheet 131d.

The substrate 131 is in a form of the flexible circuit board 131c and the reinforcement sheet 131d. The flexible circuit board 131c is used as a carrier for mounting the detection unit 132, the first flexible extension segment 1311 is used as a flexible extension part for implementing the connection between the substrate 131 and the mainboard 110, and the reinforcement sheet 131d can increase structural strength. The reinforcement sheet 131d is fastened to the electromagnetic shielding member 120, so that the detection module 130 is reliably fastened to the electromagnetic shielding member 120. The reinforcement sheet 131d may be a sheet-like body made of iron or another metal. The reinforcement sheet 131d and the flexible circuit board 131c may be connected in a manner such as adhesive bonding. A surface that is of the reinforcement sheet 131d and that faces away from the flexible circuit board 131c is used as the fifth surface 131a fastened to the electromagnetic shielding member 120. A surface that is of the flexible circuit board 131c and that faces away from the reinforcement sheet 131d is used as the sixth surface 131b on which the detection unit 132 is mounted.

When the substrate 131 has the second flexible extension segment 1312, the reinforcement sheet 131d is not disposed on the second flexible extension segment 1312, so that the second flexible extension segment 1312 is bendable, to facilitate adjusting the position and the posture of the second flexible extension segment 1312, and adjusting the second electronic element 150 on the second flexible extension segment 1312 to the predetermined position.

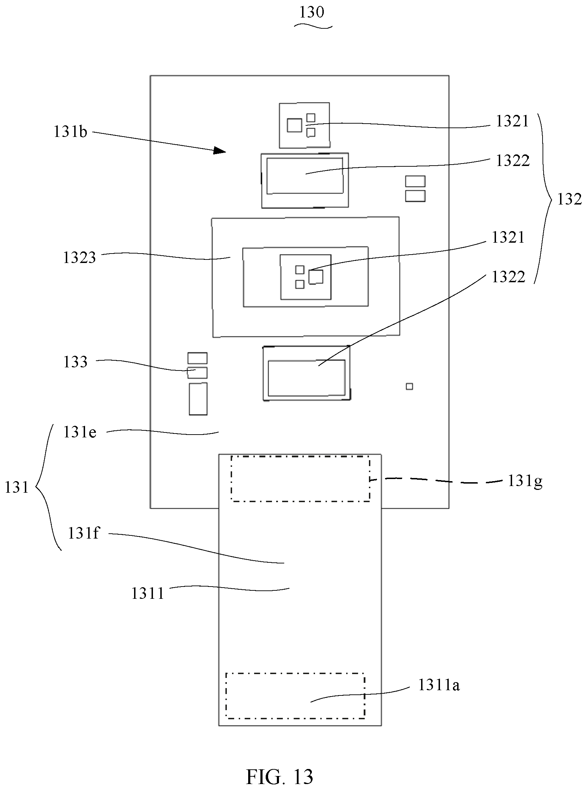

A second implementation of the substrate 131 is as follows: Refer to FIG. 13. The substrate 131 includes a first rigid board 131e and a first flexible board 131f, the first rigid board 131e is electrically connected to the first flexible board 131f, the first flexible board 131f is used as the first flexible extension segment 1311, the detection unit 132 is disposed on the first rigid board 131e, and the first rigid board 131e is fastened to the fourth surface 120b of the electromagnetic shielding member 120.

The substrate 131 is in a form of the first rigid board 131e and the first flexible board 131f. The first rigid board 131e is used as a carrier for mounting the detection unit 132, and the first flexible board 131f is used as a flexible extension part for implementing a connection between the first rigid board 131e and the mainboard 110. The first rigid board 131e and the first flexible board 131f may be electrically connected through a connector 131g, pad soldering, or in another manner. The first rigid board 131e is fastened to the electromagnetic shielding member 120, so that the detection module 130 is reliably fastened to the electromagnetic shielding member 120. A surface that is of the first rigid board 131e and that faces the electromagnetic shielding member 120 is used as the fifth surface 131a fastened to the electromagnetic shielding member 120. A surface that is of the first rigid board 131e and that faces away from the electromagnetic shielding member 120 is used as the sixth surface 131b on which detection unit 132 is mounted.

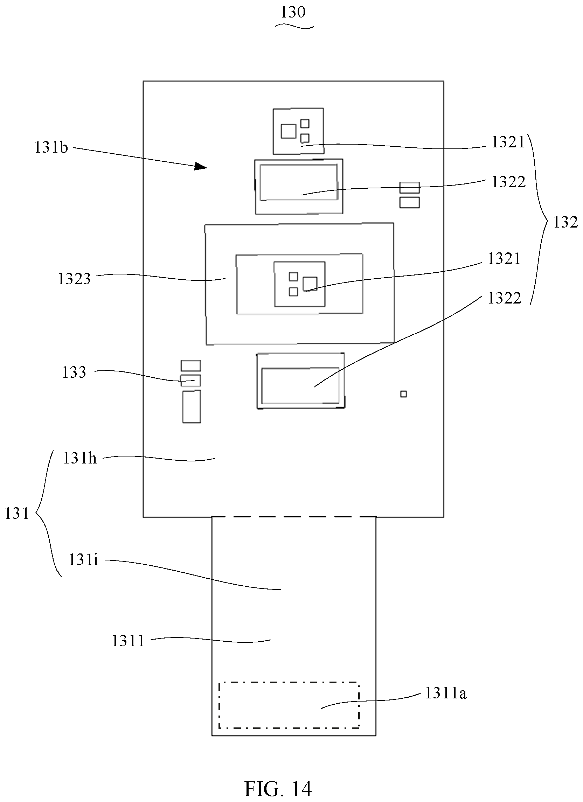

A third implementation of the substrate 131 is as follows: Refer to FIG. 14. The substrate 131 is a rigid-flexible board, the rigid-flexible board includes a first rigid portion 131h and a first flexible portion 131i, the first rigid portion 131h is electrically connected to the first flexible portion 131i, the first flexible portion 131i is used as the first flexible extension segment 1311, the detection unit 132 is disposed on the first rigid portion 131h, and the first rigid portion 131h is fastened to the fourth surface 120b of the electromagnetic shielding member 120.

The substrate 131 is in a form of the rigid-flexible board, the first rigid portion 131h and the first flexible portion 131i are of an integrated structure, the first rigid portion 131h is used as a carrier for mounting the detection unit 132, and the first flexible portion 131i is used as a flexible extension part for implementing a connection between the first rigid portion 131h and the mainboard 110. The first rigid portion 131h is fastened to the electromagnetic shielding member 120, so that the detection module 130 is reliably fastened to the electromagnetic shielding member 120. A surface that is of the first rigid portion 131h and that faces the electromagnetic shielding member 120 is used as the fifth surface 131a fastened to the electromagnetic shielding member 120. A surface that is of the first rigid portion 131h and that faces away from the electromagnetic shielding member 120 is used as the sixth surface 131b on which detection unit 132 is mounted.

When the substrate 131 is electrically connected to the mainboard 110, there are a plurality of optional implementations. The following provides two implementations as examples.

A first implementation of the electrical connection between the substrate 131 and the mainboard 110 is as follows: Refer to FIG. 8 and FIG. 11. The substrate 131 and the mainboard 110 are electrically connected through a connector 160, the connector 160 includes a first connecting portion 161 and a second connecting portion 162 that are plug-connected and fit with each other, the first connecting portion 161 is disposed on the substrate 131, and the second connecting portion 162 is disposed on the mainboard 110.

The substrate 131 and the mainboard 110 are connected through the connector 160, so that assembling is easy, and the substrate 131 is electrically connected to the mainboard 110. During assembling, the substrate 131 of the detection module 130 may be fastened to the electromagnetic shielding member 120, and the first connecting portion 161 is plug-connected to the second connecting portion 162, so that the substrate 131 is electrically connected to the mainboard 110 through the first connecting portion 161 and the second connecting portion 162.

The connector 160 may be a board-to-board (board-to-board, BTB) connector, a zero insertion force (zero insertion force, ZIF) connector, or a connector of another type. The ZIF connector does not need application of an extra insertion force during connection, and can implement a stable connection through a snap fit mechanism.

When the substrate 131 has the first flexible extension segment 1311, as shown in FIG. 11, the first connecting portion 161 may be disposed on the first flexible extension segment 1311. After the substrate 131 is fastened to the electromagnetic shielding member 120, the position of the first flexible extension segment 1311 is adjusted, so that the first connecting portion 161 and the second connecting portion 162 are plug-connected and fit with each other. In this way, assembling is convenient and reliable.

When the first flexible extension segment 1311 is not disposed on the substrate 131, the first connecting portion 161 may be disposed on a side that is of the substrate 131 and that faces the mainboard 110, and the second connecting portion 162 may be disposed on a side that is of the mainboard 110 and that faces the substrate 131. The first connecting portion 161 and the second connecting portion 162 are plug-connected and fit with each other, so that the substrate 131 is electrically connected to the mainboard 110.

A second implementation of the electrical connection between the substrate 131 and the mainboard 110 is as follows: The substrate 131 is electrically connected to the mainboard 110 through welding, the substrate 131 has a first pad (not shown in the figure), the mainboard 110 has a second pad (not shown in the figure), and the first pad and the second pad are welded.

The substrate 131 and the mainboard 110 are connected through the pads, so that assembling is easy, and the substrate 131 is electrically connected to the mainboard 110. During assembling, the substrate 131 of the detection module 130 may be fastened to the electromagnetic shielding member 120, and the first pad and the second pad are welded, so that the substrate 131 is electrically connected to the mainboard 110 through the first pad and the second pad.

When the detection unit 132 is disposed, the detection unit 132 may include at least one of a photoplethysmography sensor, an electrocardiogram sensor, and a temperature sensor. In other words, the detection unit 132 with one or more functions may be configured according to requirements. Different detection units 132 may monitor different physiological parameters of the user.

The photoplethysmography (photoplethysmography, PPG) sensor uses a photoelectric sensor to detect an intensity of light reflected after absorption by human blood and tissue, record changes in blood vessel volume during a cardiac cycle, and determine heart rate and blood oxygen from an obtained pulse waveform.

The electrocardiogram (electrocardiogram, ECG) sensor uses electrodes in contact with human skin to record timing and intensities of electrical signals that cause heart beats.

The temperature sensor uses a temperature-sensitive element to measure a temperature change of a human body.

When the detection unit 132 includes the photoplethysmography sensor, as shown in FIG. 11 to FIG. 14, the photoplethysmography sensor includes one or more light emitting diodes 1321 (light emitting diodes, LEDs) and one or more photoelectric diodes 1322 (photoelectric diodes, PDs), a light emitting surface of the light emitting diode 1321 is disposed facing away from the mainboard 110, and a light receiving surface of the photoelectric diode 1322 is disposed facing away from the mainboard 110.

The light emitting diode 1321 is a semiconductor light emitting component that emits light. The photoelectric diode 1322 is a semiconductor photoelectric detection component that converts light into an electrical signal. The light emitting diode 1321 and the photoelectric diode 1322 are disposed on the substrate 131, and are electrically connected to the substrate 131. During operation, a part of an optical signal sent by the light emitting diode 1321 to the human body is absorbed by human blood and tissue, and a part of the optical signal is reflected and received by the photoelectric diode 1322. A change in blood vessel volume during a cardiac cycle is recorded based on a collected optical signal, and a heart rate and blood oxygen are obtained from an obtained pulse waveform. A combination and a layout of the light emitting diode 1321 and the photoelectric diode 1322 may be determined based on an optical path design.

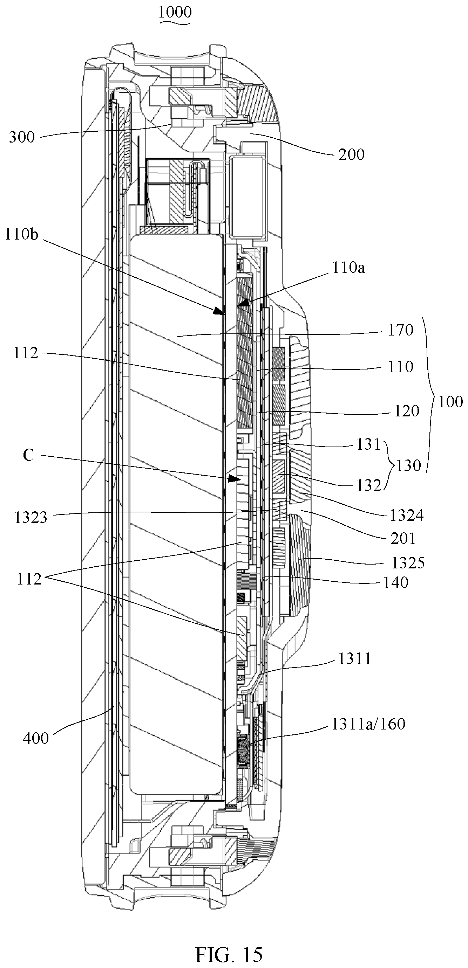

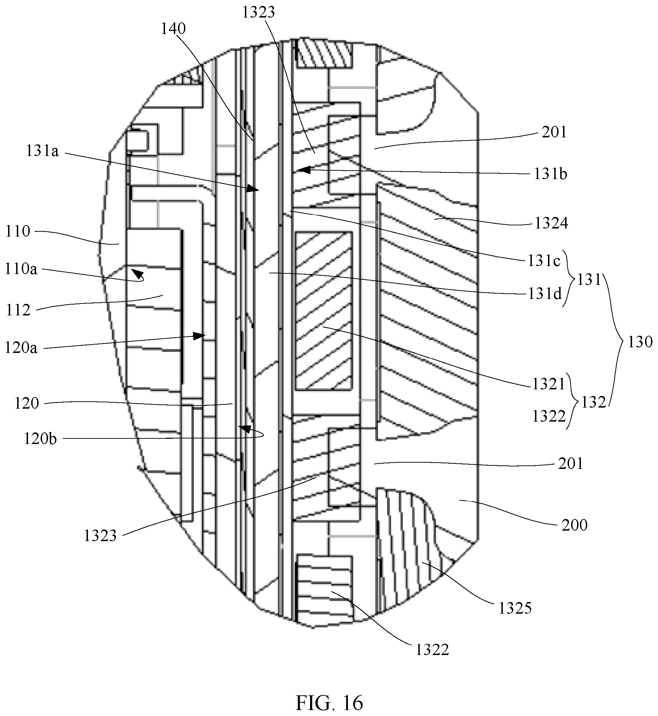

Refer to FIG. 15 and FIG. 16. When the detection unit 132 of the detection module 130 is the PPG sensor, the circuit board assembly 100 having the mainboard 110, the electromagnetic shielding member 120, and the detection module 130 is directly disposed on the middle frame 300, and a predetermined gap needs to be provided between the mainboard 110 and the bottom housing 200, so that a predetermined distance exists between the PPG sensor and the bottom housing 200. In this way, the PPG sensor can operate normally. To be specific, the light emitting diode 1321 in the PPG sensor emits an optical signal to the human body, and the photoelectric diode 1322 in the PPG sensor receives an optical signal reflected by the human body, so that a design of an optical path of the PPG sensor is optimized.

In some embodiments, as shown in FIG. 12, a light shielding member 1323 is disposed between the light emitting diode 1321 and the photoelectric diode 1322, to prevent light of the light emitting diode 1321 from directly irradiating on the photoelectric diode 1322. The light shielding member 1323 may be used to prevent the light of the light emitting diode 1321 from directly irradiating on the photoelectric diode 1322, so that the photoelectric diode 1322 receives the optical signal reflected by the human body, thereby improving detection accuracy.

The light shielding member 1323 may be made of a material such as foam. The light shielding member 1323 may be set to be in a shape of a rectangle, a circle, or the like, and is disposed around the light emitting diode 1321. An area of the light shielding member 1323 is set according to requirements. The light shielding member 1323 may be fastened to the substrate 131 in a manner of bonding or the like.

When the bottom housing 200 and the middle frame 300 are assembled, as shown in FIG. 15 and FIG. 16, an inner wall of the bottom housing 200 may have a separation rib 201. In a process in which the bottom housing 200 is fastened to the middle frame 300, the separation rib 201 abuts against the light shielding member 1323, to implement light shielding effect between the light emitting diode 1321 and the photoelectric diode 1322.

For example, as shown in FIG. 12, two photoelectric diodes 1322, two photoelectric diodes 1322, and one frame-shaped light shielding member 1323 are disposed on the substrate 131. The photoelectric diodes 1322 and the photoelectric diodes 1322 are arranged in a staggered manner in a predetermined direction, and the light shielding member 1323 is disposed between the two adjacent photoelectric diodes 1322 and is disposed around one photoelectric diode 1322.

In some embodiments, as shown in FIG. 15 and FIG. 16, a first lens 1324 is disposed in front of the light emitting surface of the light emitting diode 1321, and a second lens 1325 is disposed in front of the light receiving surface of the photoelectric diode 1322, to implement better light emitting and light receiving of the PPG sensor. The light emitting diode 1321 is combined with the first lens 1324, so that light generated by the light emitting diode 1321 is well emitted. The photoelectric diode 1322 is combined with the second lens 1325, so that external light is well received by the photoelectric diode 1322. In addition, a part of the light emitting diodes 1321 and a part of the photoelectric diodes 1322 are adjacently disposed, and may share a same lens.

In some embodiments, as shown in FIG. 6, FIG. 8, and FIG. 10, the battery 170 is fastened to the second surface 110b of the mainboard 110, and the battery 170 is electrically connected to the mainboard 110, to enable the circuit board assembly 100 to have a power supply capability. The battery 170 may be configured to supply power to components such as the mainboard 110 and the detection module 130. The mainboard 110 is used as a single board structure, and the battery 170 is disposed on the second surface 110b of the mainboard 110, so that a size and a capacity of the battery 170 can be large, increasing maximum operating time of the entire device. The battery 170 may be disposed in the middle frame 300. The battery 170 may be fastened to the mainboard 110 through adhesive or in another manner.

When the circuit board assembly 100 and the electronic device 1000 in this embodiment need to be confirmed, the device may be disassembled, to see whether the detection module 130 is fastened to the electromagnetic shielding member 120. Alternatively, a tomographic diagram of the circuit board assembly 100 may be obtained through computed tomography (computed tomography, CT) for determining whether the detection module 130 is fastened to the electromagnetic shielding member 120.

In conclusion, the foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A circuit board assembly, comprising a mainboard, an electromagnetic shielding member, and a detection module, wherein
the mainboard has a first surface and a second surface that face away from each other;
the electromagnetic shielding member has a third surface and a fourth surface that face away from each other, the electromagnetic shielding member is disposed on the first surface of the mainboard, and the third surface of the electromagnetic shielding member and the first surface of the mainboard are disposed facing each other; and
the detection module comprises a substrate and a detection unit, the substrate has a fifth surface and a sixth surface that face away from each other, the fifth surface of the substrate is fastened to the fourth surface of the electromagnetic shielding member, the detection unit is mounted on the sixth surface of the substrate, and the substrate is electrically connected to the mainboard.

2. The circuit board assembly according to claim 1, wherein the fifth surface of the substrate is bonded through adhesive, welded, riveted, snap-fitted, or connected through a fastener to the fourth surface of the electromagnetic shielding member.

3. The circuit board assembly according to claim 1 or 2, wherein a first electronic element is disposed on the first surface of the mainboard, and the electromagnetic shielding member covers the first electronic element; and
the electromagnetic shielding member comprises a plate-shaped portion and a flanged portion, the flanged portion is connected to an edge of the plate-shaped portion, the flanged portion is fastened to the first surface of the mainboard, an end face that is of the plate-shaped portion and that faces the mainboard forms the third surface, and an end face that is of the plate-shaped portion and that faces away from the mainboard forms the fourth surface.

4. The circuit board assembly according to any one of claims 1 to 3, wherein the substrate has a first flexible extension segment, and the first flexible extension segment is electrically connected to the mainboard.

5. The circuit board assembly according to claim 4, wherein a connection position between the first flexible extension segment and the mainboard is located on the first surface of the mainboard, and is disposed close to an edge of the electromagnetic shielding member.

6. The circuit board assembly according to claim 4 or 5, wherein the substrate comprises a flexible circuit board and a reinforcement sheet, the flexible circuit board and the reinforcement sheet are stacked, the first flexible extension segment is formed on the flexible circuit board, the reinforcement sheet is not disposed on the first flexible extension segment, the detection unit is disposed in a region that is of the flexible circuit board and that corresponds to the reinforcement sheet, and the reinforcement sheet is fastened on the fourth surface of the electromagnetic shielding member.

7. The circuit board assembly according to any one of claims 1 to 6, wherein the substrate is electrically connected to the mainboard through a connector, the connector comprises a first connecting portion and a second connecting portion that are plug-connected and fit with each other, the first connecting portion is disposed on the substrate, and the second connecting portion is disposed on the mainboard; or
the substrate is electrically connected to the mainboard through welding, the substrate has a first pad, the mainboard has a second pad, and the first pad is welded to the second pad.

8. The circuit board assembly according to any one of claims 1 to 7, wherein the detection unit comprises at least one of a photoplethysmography sensor, an electrocardiogram sensor, and a temperature sensor.

9. An electronic device, comprising a bottom housing, a middle frame, and the circuit board assembly according to any one of claims 1 to 8, wherein the bottom housing is connected to the middle frame, the mainboard is disposed on the middle frame, the first surface of the mainboard and the bottom housing are disposed facing each other, and the detection module is disposed close to the bottom housing.

10. The electronic device according to claim 9, further comprising a screen assembly, wherein the screen assembly is disposed on the middle frame, the screen assembly is electrically connected to the mainboard, and the second surface of the mainboard and the screen assembly are disposed facing each other.

11. The electronic device according to claim 10, wherein the electronic device comprises a watch or a band.
